(19) Europäisches Patentamt
European Patent Office
Office européen des brevets

(11) EP 4 559 885 A1

(12) EUROPEAN PATENT APPLICATION

(43) Date of publication:
**28.05.2025 Bulletin 2025/22**

(21) Application number: **23212125.1**

(22) Date of filing: **24.11.2023**

(51) International Patent Classification (IPC):
***C04B 20/02** (2006.01)* ***C04B 28/02** (2006.01)*
***C04B 40/00** (2006.01)* ***G01N 33/38** (2006.01)*
***G06Q 50/08** (2012.01)*

(52) Cooperative Patent Classification (CPC):
(C-Sets available)
**C04B 28/02; C04B 20/026; C04B 40/0039;**
**G06Q 10/063; G06Q 50/04; G06Q 50/08;**
G01N 33/383 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Sika Technology AG**
**6340 Baar (CH)**

(72) Inventors:
- **TAMURA, Atsushi**
  **Hiratsuka, 254-0021 (JP)**
- **ODA, Kenta**
  **Hiratsuka, 254-0021 (JP)**
- **ONODA, Noriyuki**
  **Hiratsuka, 254-0021 (JP)**
- **SHINDO, Kenji**
  **Hiratsuka, 254-0021 (JP)**

(74) Representative: **Sika Patent Attorneys**
**C/o Sika Technology AG**
**Corp. IP Dept.**
**Tüffenwies 16**
**8048 Zürich (CH)**

# (54) METHODS FOR THE CONTROLLED INTRODUCTION OF AIR IN A CEMENTITIOUS MATERIAL COMPRISING FLY ASH

(57) This invention relates to methods for the controlled introduction of air in a cementitious material comprising fly ash where a polymeric dispersant, at least one air-entraining agent, at least one anti-foam agent, and water is intergrinded and/or intermixed. This invention also relates to computer implemented methods to determine proportions of admixture constituents to be added for the controlled introduction of air to a cementitious material comprising fly ash.

EP 4 559 885 A1

(52) Cooperative Patent Classification (CPC): (Cont.)

C-Sets
**C04B 20/026, C04B 24/302, C04B 2103/302, C04B 2103/50;**
**C04B 28/02, C04B 18/08, C04B 2103/408, C04B 2103/302, C04B 2103/50;**
**C04B 40/0039, C04B 24/246, C04B 2103/302, C04B 2103/50, C04B 2103/50**

# Description

## Technical Field

This invention relates to methods for the controlled introduction of air in a cementitious material comprising fly ash. This invention also relates to computer implemented methods to determine proportions of admixture constituents to be added for the controlled introduction of air to a cementitious material comprising fly ash.

## Background of the invention

In recent years fly ash, an industrial by-product, has been actively used in cementitious materials in the civil engineering and construction industries to make effective use of resources and reduce environmental impact.

However, when fly ash was used as part of the binder system, the stability of the resulting cementitious composition was sometimes reduced. In order to improve workability and other properties of cementitious compositions such as concrete compositions, it is known to use admixtures. Recently, it is also known to use phenolic resin-based dispersants made by polycondensation of several kinds of phenolic comonomers and aldehydes.

For example, WO2018/147378 proposes an admixture containing a phenolic resin-based dispersant made by polycondensation of multiple phenolic comonomers and aldehydes and a polycarboxylic acid-based polymer.

In particular when fly ash is used as a cementitious binder, it can be difficult to introduce and/or maintain a desired content of air in a cementitious material. This is especially the case when a relatively high percentage of fly ash is present.

There is thus a need to provide suitable methods for the controlled introduction of air in a cementitious material comprising fly ash.

## Summary of the invention

It is an objective of the present invention to provide methods for the controlled introduction of air in a cementitious material comprising fly ash. In particular, it is an objective of the present invention to provide methods employing suitable admixtures for the controlled introduction of air in a cementitious material comprising fly ash.

If is a further objective of the present invention to provide methods for the determination of admixtures, especially the determination of proportions of admixture constituents, suitable for the controlled introduction of air in a cementitious material comprising fly ash.

Surprisingly, it has been found that a mixture of a polymeric dispersant, at least one air-entraining agent, at least one anti-foam agent, and water is suitable for the controlled introduction of air in a cementitious material comprising fly ash.

Therefore, the objective of the present invention is solved by the subject matter of claim 1.

Further aspects of the invention are the subject-matter of independent claims. Preferred embodiments are the subject-matter of dependent claims.

## Detailed ways

In a first aspect the present invention relates to a method for the controlled introduction of air in a cementitious material comprising a cementitious binder and fly ash, said method comprising the steps of:

i) providing a cementitious material comprising fly ash,
ii) providing an admixture comprising or consisting of a polymeric dispersant, at least one air-entraining agent, at least one anti-foam agent, and water, and
ii) intergrinding and/or intermixing said cementitious material and said admixture.

The term "controlled introduction of air" within the present context is synonymous to "stabilization of air" or "stabilization of air content". A controlled introduction of air can mean that a specified amount of air is introduced into a cementitious material during preparation thereof, preferably during the mixing of a dry cementitious material with mixing water. A controlled introduction of air can mean that air is distributed in a cementitious material in a specified way, especially homogeneously and with a certain size and spacing of air pores. Thus, preferably, the controlled introduction of air means that a specified amount of air and/or air pores with specified size and spacing is introduced into a cementitious material during preparation thereof.

The content of air is preferably given in Vol-% relative to the total volume of the cementitious material. The content of air can for example be measured according to standard JIS A 1128:2019. The size of air pores can for example be measured by mercury porosimetry and gas adsorption as described in standard ISO 15901-1:2019 and ISO 15901-2:2022. The size of air pores and the spacing of air pores can also be measured by preparing thin sections of the cured binder compositions and imaging them by scanning electron microscopy, in particular at 200x magnification. The pore size and pore spacing can then be determined from the scanning electron microscope images. If the pores are not completely circular, the longest diameter is taken as the pore diameter or pore size.

In particular, the amount of air introduced into a cementitious material by a method of the present invention is between 1 - 20 Vol-%, preferably between 3-10 Vol-%, more preferably between 4-8 Vol-%, relative to the total volume of the cementitious material.

A cementitious composition within the present

context is a composition comprising at least one cementitious binder as well as fly ash, and optionally one or more of aggregates, fillers, admixtures, and water.

The cementitious binder is chemically different from fly ash. In particular, the cementitious binder is chosen from cement, gypsum, lime, pozzolane, and/or latent hydraulic material, Preferably, the cementitious binder is chosen from Portland cement, aluminate cement, calcium sulfoaluminate cement. A particularly preferred cementitious binder is Portland cement according to standard EN 197-1:2011 or JIS R 5210:2009. It is also possible that the cementitious binder is a mixture of two or more of cement, gypsum, lime, pozzolane, and/or latent hydraulic material. In particular, the cementitious binder consists of Portland cement and fly ash.

Fly ash is a fine powder that is a byproduct of burning pulverized coal. Fly ash is a pozzolan. It comprises aluminous and siliceous material. In particular, in a method of the present invention, the fly ash is according to standard JIS 6201:2015. However, fly ash according to other standard, such as ASTM C618-22 or EN 450-1:2012 is also suitable.

According to embodiments, fly ash of the present invention has a content of unburnt carbon of between 0.01 - 12.0 w%, preferably 0.1 - 10.0 w%, more preferably 0.1 - 6.0 w%, relative to the total dry weight of the fly ash.

Preferably, the methylene blue absorption of fly ash of the present invention is between 0.1 - 2 mg/g, more preferably between 0.15 - 0.5 mg/g. The Blaine fineness of suitable fly ash can, for example, be between 1900 - 5000 $cm^2/g$.

A cementitious composition of the present invention comprises a cementitious binder and fly ash. It is preferred, that the content of fly ash in the cementitious material is not more than 70 w%, preferably not more than 50 w%, more preferably not more than 30 w%, especially not more than 20 w% relative to the total dry weight of the cementitious material. It is likewise preferred, that the content of fly ash in the cementitious material is not less than 1 w%, preferably not less than 5 w%, more preferably not less than 10 w%, relative to the total dry weight of the cementitious binder.

The mass ratio of fly ash to cementitious binder may be 1:20 to 1:1, preferably 1:10 to 1:2.

A cementitious composition of the present invention may additionally comprise one or more of aggregates, fillers, additives, and water. Aggregates and fillers are not particularly limited and are known to the skilled person per se. Additives are not polymeric dispersants, air-entraining agents, and anti-foam agents as defined within the present context. For example, additives can be superplasticizers such as polycarboxylate ethers or lignosulphonates, accelerators, retarders, strength enhancers, thickening agents, shrinkage reducers, fibers, redispersible polymer powders, corrosion inhibitors, pigments, biocides, or mixtures thereof.

The polymeric dispersant used in a method of the present invention preferably is a phenol resin-based dispersant obtained from the polycondensation of the following monomers (i) to (v):

(i) a mol% of monomers of formula (I)

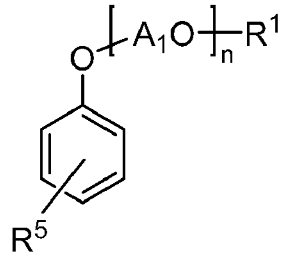

(I),

(ii) b mol% of monomers of formula (II)

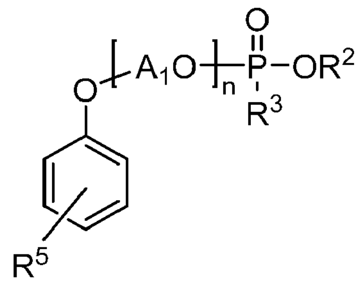

(II),

(iii) c mol% of monomers of formula (III)

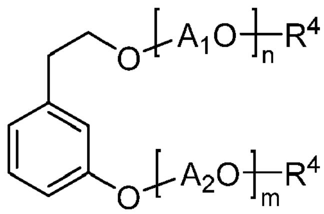

(III),

and

(iv) d mol% of monomers of formula (IV)

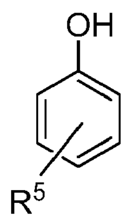

(IV),

(v) e mol% of monomers selected from aldehydes, preferably formaldehyde, metaformaldehyde, paraformaldehyde, formalin, or mixtures thereof, wherein

$R^1$ is H, a C1 - C4 alkyl group, or a C2 - C5 acyl group,

$R^2$ is H, an alkali metal or an alkaline earth metal ion, or a moiety of structure

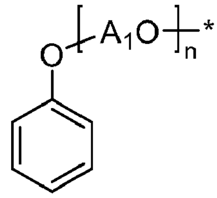

,

$R^3$ is H or is O-$R^2$,

$R^4$ is H, a C1 - C4 alkyl group, or a phosphate ester group,

$R^5$ is H, a C1 - C18 alkyl group, a polyisobutylene moiety, or a sulfonic acid moiety,

$A_1$ and $A_2$ independently of one another are $C_xH_{2x}$ group with x = 2 to 5,

n = 1 - 350, m = 2 - 300,

a molar ratio of a : b : c : d is 0.1 - 2.0 : 0.1 - 6.0 : 0.1 - 2.0 : 0.0 - 0.5, and

a molar ratio of (a + b + c + d) : e is from 1 : 10 to 10 : 1.

Monomer (i) is a compound where an alkylene oxide with 2 to 4 carbon atoms has been added to phenol or a substituted phenol, and also includes derivatives (alkyl esters or fatty acid esters) of such alkylene oxide adducts. Monomer (ii) is a phosphate ester derivative of a compound to which an alkylene oxide with 2 to 4 carbon atoms has been added to phenol or its substituent. Monomer (iii) is a compound in which an alkylene oxide is added to two hydroxy groups of hydroxyethylphenol, and also includes derivatives of alkylene oxide adducts such as phosphate esters.

The C1-C4 alkyl groups in $R^1$ include methyl, ethyl, propyl, and butyl groups, which can be branched (e.g., isopropyl, isobutyl, sec-butyl, tert-butyl) and/or cyclic (e.g., cyclopropyl, isobutyl, sec-butyl, tert-butyl) structures. C1-C5 acyl groups in $R^1$ include saturated or unsaturated acyl groups (R'(CO)- group, R' being a hydrocarbon group with 1-4 carbon atoms). For example, $R^1$ can be an acyl group derived from acetic acid, propionic acid, butanoic acid, and pentanoic acid. C1-C18 alkyl groups in $R^5$ include methyl, ethyl, propyl, butyl, pentyl, hexyl, heptyl, octyl, nonyl, decyl, dodecyl (lauryl), tetradecyl (myristyl), hexadecyl (palmityl), octadecyl. These can be branched (e.g., isopropyl, isobutyl, sec-butyl, tert-butyl, neopentyl, etc.) and/or cyclic (e.g., cyclopropyl, cyclopentyl, cyclohexyl, 1,2,3,4,5,6,7), cyclohexyl group, 1-adamantyl group, etc.).

Alkylene oxide (-$A_1$O-) has an alkylene group $A_1$ of the chemical formula $C_xH_{2x}$ with x = 2-5. Thus, the alkylene oxide may be ethylene oxide, propylene oxide, butylene oxide, and pentene oxide, and these alkylene oxides may be single or mixed. When two or more alkylene oxides are mixed-added, they may be arranged in random maner or blockwise or with a gradient.

According to embodiments, at least some of the alkylene groups $A_1$ of the alkylene oxide (-$A_1$O-), such as 1 mol% or more, preferably 1 mol% to 20 mol%, more preferably 3 mol% to 20 mol%, have 3 to 5 carbons (x = 3 to 5). The percentage may be less than 100 mol%, preferably less than 90 mol%, more preferably less than 70 mol%, still more preferably less than 50 mol%, especially less than 20 mol%. When at least some of the alkylene groups have 3 to 5 carbons, the resulting phenolic resin-based dispersant can be made relatively hydrophobic, thereby enabling the use of fly ash, especially when a relatively high percentage of fly ash is used in the cementitious composition.

According to embodiments, the alkylene groups $A_1$ of the alkylene oxide (-$A_1$O-) may all have 2 carbons (x=2), i.e., all may be ethylene.

n is the average number of moles of alkylene oxide added, and is a number from 1 to 350, preferably 1 to 300, more preferably 3 to 150.

The hydroxyethylphenol moiety in monomer (III) can be o-hydroxyethyl-phenol, m-hydroxyethyl-phenol, or p-hydroxyethyl-phenol. Preferably, monomer (iii) is a compound in which an alkylene oxide with 2 to 5 carbon atoms is added to o-hydroxyethyl-phenol. By anionizing the end of monomer (iii), i.e., making it a phosphate ester derivative, the kneading time can be reduced when it is added to a wet cementitious composition.

The molar ratio of monomers (i) to (iv), i.e., a:b:c:d, may be 0.1 - 2.0 : 0.1 - 6.0 : 0.1 - 2.0 : 0.0 - 0.5. In other words, monomer (iv) is not an essential component, and this monomer may not be used. The molar ratio of a: b: c: d may be 0.5 to 1.5: 0.5 to 6.0: 0.1 to 1.0: 0.0 to 0.5, 0.5 to 1.5: 0.5 to 6.0: 0.1 to 1.0: 0.0, or 0.1 to 2.0: 0.1 to 6.0: 0.1 to 2.0: 0.0. The molar ratio of monomer (v) to the sum of monomers (i)-(iv), (a+b+c+d):e may be 1:10 to 10:1. This ratio may also be 2:10 to 10:2 or 3:10 to 10:3.

The polymerization method for obtaining the above phenolic resin-based dispersant by polycondensation of the above monomers is not particularly limited.

In polycondensation, the order and method of addition of each monomer are not particularly limited. For example, all monomers to be reacted can be added in a batch before the polycondensation reaction, a portion of the monomers to be used can be added before the polycondensation reaction, and then the remainder can be added dropwise, or a portion of the monomers to be used can be added before the polycondensation reaction, and after a certain reaction time has passed, the remaining After a certain reaction time, the remaining monomers can be added additionally.

Polycondensates are obtained, for example, by polycondensation of the monomers used in the presence of a dehydration catalyst, either solvent-free or under solvent, at a reaction temperature of 80°C to 150°C and under ambient to increased pressure, for example, 1013 mbar or increased pressure by 100 to 10000 mbar.

The above mentioned dehydration catalysts include hydrochloric acid, perchloric acid, nitric acid, formic acid, methanesulfonic acid, octyl sulfonic acid, dodecyl sulfonic acid, vinyl sulfonic acid, allylsulfonic acid, phenol sulfonic acid, acetic acid, sulfuric acid, diethyl sulfate, dimethyl sulfate, phosphoric acid, oxalic acid, boric acid, benzoic acid, phthalic acid, Salicylic acid, pyruvic acid, maleic acid, malonic acid, nitrobenzoic acid, nitrosalicylic acid, paratoluenesulfonic acid, benzenesulfonic acid, dodecylbenzenesulfonic acid, trifluoromethanesulfonic acid, fluoroacetic acid, thioglycolic acid, mercaptopropionic acid, activated white earth, etc., One or more of these dehydration catalysts can be used alone or in combination.

When the polycondensation reaction is carried out under solvent, water, glycol ether compounds such as propylene glycol monomethyl ether (PGME), aromatic compounds such as toluene and xylene, cyclic aliphatic compounds such as methylcyclohexane can be used as solvents. Acetic acid, for example, can also be used as a solvent.

The reaction can preferably be carried out at a temperature of 95°C to 130°C, and the polycondensation reaction can be completed by reacting for 3 to 25 hours.

The polycondensation reaction is preferably carried out under acidic conditions, and preferably the pH of the reaction system should be 4 or less.

In addition to the above monomers, other monomers capable of polycondensation with these compounds may be blended into the monomer mixture to the extent that the effect of the invention is not impaired.

After completion of the polycondensation reaction, various known methods can be employed to reduce the content of unreacted monomer (v). For example, the pH of the reaction system can be made alkaline and heat-treated to 60 to 140°C, the reaction system can be depressurized (e.g., gauge pressure of -0.1 to -0.001 MPa) to volatilize and remove unreacted monomer (v), and even small amounts of sodium hydrogen sulfite, ethylene urea, and/or polyethyleneimine can be added.

The dehydration catalyst used in the reaction can be neutralized after completion of the reaction and removed by filtration as a salt form, but even if the catalyst is not removed, the performance of the polymer as a dispersing agent is not impaired. In addition to the above filtration, other methods of catalyst removal can be used including phase separation, dialysis, ultrafiltration, and the use of ion exchangers.

By neutralizing and diluting the reactants with water, the weighing and other operations for use as a dispersant is improved. Alkaline hydroxides such as sodium hydroxide and potassium hydroxide, alkaline earth hydroxides such as calcium hydroxide, ammonia, organic amines such as monoethanolamine, diethanolamine, and triethanolamine are examples of basic compounds used for neutralization.

The phenolic resin dispersant finally obtained preferably is in the range of 1000 to 50000 g/mol, more preferably 1000 to 30000 g/mol, still more preferably 1000 to 10000 g/mol, especially 2000 to 9000 g/mol by weight average molecular weight as measured by gel permeation chromatography method against a polyethylene glycol standard.

According to embodiments, an air-entraining agent according to the present invention is selected from anionic or zwitterionic surfactants. Suitable anionic surfactants are diphenylene oxide sulfonates, alkane and alkylbenzene sulfonates, alkylnaphthalene sulfonates, olefin sulfonates, alkyl ether sulfonates, alkyl sulfates, alkyl ether sulfates, alpha-sulfo fatty acid esters, acylaminoalkane sulfonates, acylisothionates, alkyl ether carboxylates, N-acyl sarcosinates, alkyl and alkyl ether phosphates. Examples of suitable anionic surfactants include C8-C18-alkyl sulfates, C8-C18-alkyl ether sulfates, C8-C18-alkyl sulfonates, C8-C18-alkyl benzene sulfonates, C8-C18-$\alpha$-olefin sulfonates, C8-C18-sulfosuccinates, $\alpha$ -sulfo-C8-C18-fatty acid salts and C8-C18-fatty acid salts. The anionic surfactants are generally present as alkali metal salts, particularly as sodium salts. Examples of sodium salts of anionic surfactants are sodium lauryl sulfate, sodium myristyl sulfate, sodium cetyl sulfate, sodium sulfates of ethoxylated lauryl or myristyl alcohol with a degree of ethoxylation of 2 to 10, lauryl or cetyl sulfonate sodium salt, hexadecyl benzene sulfonate sodium salt, C14/C16-$\alpha$-olefin sulfonate sodium salt, lauryl or cetyl sulfosuccinate sodium salt, disodium 2-sulfolaurate, sodium stearate or mixtures thereof.

Suitable zwitterionic surfactants are betaines. One example of zwitterionic surfactants is cocamidopropyl betaine and particularly lauramidopropyl betaine.

Air-entraining agent may also be selected from protein based surfactants, root resins, rosin salts, and tall oil. Both, plant and animal proteins or mixtures thereof can be used as protein based surfactants. Examples of proteins suitable as surfactants include keratin, hydrolyzed keratin, collagen, hydrolyzed collagen, or soy-based proteins.

Mixtures of two or more air-entraining agents can also be used.

According to embodiments, an anti-foam agent according to the present invention is selected from non-ionic surfactants. Suitable non-ionic surfactants are alkylphenol polyglycol ethers, fatty alcohol polyglycol ethers, fatty acid polyglycol ethers, fatty acid alkanolamides, block copolymers, amine oxides, glycerol fatty acid esters, sorbitan esters or alkylpolyglucosides. Examples of suitable nonionic surfactants include C8-C24-fatty alcohol ethoxylates, block copolymers of ethylene oxide and propylene oxide, C8-C24-alkyl polyglycosides, and polyalkoxylated sugar alcohols, especially polyalkoxylated sorbitol.

Mixtures of two or more anti-foam agents can also be used.

An admixture of the present invention comprises or consists of a polymeric dispersant, an air-entraining agent, an anti-foam agent, as described above, and water. It is possible, that an admixture of the present invention comprises further constituents such as, for example, surfactants, biocides, extenders, or pigments. Extenders can especially be organic solvents and/or inert fillers.

The admixture of the present invention may be a mono-component admixture. This means that all constituents of the admixture a present in one compartment. A mono-component admixture has the advantage that dosing is particularly simple.

The admixture of the present invention may be a two-component or multi-component admixture. This means, that individual constituents of the admixture are present in two or more spatially separated containers. A two-component or multi-component admixture has the advantage that shelf life may be longer and that adjustment of dosages of individual components is possible.

According to embodiments, the admixture of the present invention is intermixed with a cementitious material of the present invention. Thereby, it is possible to add the admixture to a dry cementitious material together with any constituent of said dry cementitious material. It is likewise possible to add the admixture together with or shortly after the mixing water. In case of a two-component or multi-component admixture, it is possible to intermix the individual components at different point of time with the cementitious material. It is, for example, possible to intermix one or more components of the admixture with a dry mix and add further components together with the mixing water.

Tools for intermixing the cementitious material and the admixture are not particularly limited. Suitable tools include hand held agitator, Hobart mixer, portable concrete mixer, mixing truck, mixing bucket, paddle mixer, jet mixer, screw mixer, auger mixer, horizontal single shaft mixer, twin shaft paddle mixer, vertical shaft mixer, planetary mixer, ribbon blender, orbiting mixer, change-can mixer, tumbling vessel, vertical agitated chamber or air agitated operations. Intermixing can be continuously, semi-continuously or batch-wise.

According to embodiments, the admixture is of the present invention is intergrinded with a cementitious material of the present invention. In particular, the admixture is of the present invention is intergrinded with at least one constituent of the cementitious material of the present invention. It can be especially preferred to intergrind the admixture of the present invention with cement and/or fly ash.

Tools for intergrinding are not particularly limited. It is, for example, possible to intergrind the admixture and at least one constituent of the cementitious material preferably cement or fly ash, in a ball mill or vertical roller mill.

According to embodiments, in a method of the present invention, the admixture is intergrinded and/or is intermixed with the cementitious material in an amount so that the following weight ratios result (in each case relative to the total weight of cementitious binder contained)

- 0.1 - 5 w%, preferably 0.5 - 3 w%, especially 1 - 2 w%, of the polymeric dispersant,
- 0.001 - 0.03 w%, preferably 0.005 - 0.02 w%, especially 0.01 - 0.015 w%, of the at least one air-entraining agent,
- 0.01 - 0.5 w%, preferably 0.02 - 0.25 w%, especially 0.1 - 0.2 w%, of the at least one anti-foam agent.

According to embodiments, the cementitious material of the present invention contains an amount of air between 1 - 20 Vol-%, preferably between 3 - 10 Vol-%, more preferably between 4-8 Vol-% relative to the total volume of the cementitious material at a time $t_0$ after mixing with water. The content of air can for example be measured according to standard JIS A 1128:2019. The time $t_0$ after mixing with water preferably is 20 minutes or less, more preferably is 10 minutes or less, in particular is 5 minutes or 3 minutes.

In another aspect, the present invention relates to a cementitious material obtained by a method as described above.

All embodiments and features as described above also apply to this aspect. In particular, the cementitious material is a concrete, a dry mortar, or a wet mortar.

According to embodiments, the cementitious material of the present invention comprises

(a) 200 to 700 kg/m$^3$ of cementitious binder and fly ash,

(b) 700-950 kg/m3 of fine aggregate, preferably sand, and

(c) 800-1100 kg/m3 coarse aggregate, preferably gravel.

A mass ratio of water to the sum of cementitious binder and fly ash preferably is between 0.25 and 0.6

According to embodiments, the cementitious material of the present invention contains an amount of air between 1 - 20 Vol-%, preferably between 3 - 10 Vol-%, more preferably between 4-8 Vol-% relative to the total volume of the cementitious material at a time $t_0$ after mixing with water. The content of air can for example be measured according to standard JIS A 1128:2019. The time $t_0$ after mixing with water preferably is 20 minutes or less, more preferably is 10 minutes or less, still more preferably is 3 minutes or less, especially is 0 minutes.

According to embodiments, the cementitious material of the present invention contains an amount of air at a time $t_1$ after mixing with water that differs by not more than 100%, preferably not more than 33%, more preferably not more than 20%, especially not more than 10%, from the amount of air at a time $t_0$ after mixing with water, where the time $t_1$ is later than the time $t_0$. In

particular, the time $t_0$ is 0 minutes and the time $t_1$ is 80 minutes, or is 60 minutes, or is 40 minutes, or is 20 minutes.

In another aspect the present invention relates to an admixture for use in a method as described above, said admixture comprising (in each case relative to the total weight of the admixture):

a) 1 - 20 w%, preferably 5 - 15 w%, more preferably 8 - 13 w%, of the polymeric dispersant,
b) 1 - 20 w% of at least one air-entraining agent,
c) 0.1 - 10 w% of at least one anti-foaming agent, and
d) the remainder to 100 w% of water.

All embodiments and features as described above also apply to this aspect.

In particular, an admixture of the present invention may be a mono-component or may be a two-component or multi-component admixture as described above.

It is possible, that an admixture of the present invention comprises further constituents such as, for example, surfactants, biocides, extenders, or pigments. Extenders can especially be organic solvents and/or inert fillers.

In another aspect, the present invention relates to a computer implemented method to determine the respective proportions of at least one admixture constituent, preferably at least two admixture constituents, more preferably at least three admixture constituents, in particular four admixture constituents to be added for a controlled introduction of air to a cementitious material comprising a cementitious binder and fly ash, the method comprising the steps of:

a) inputting a desired controlled introduction of air in the cementitious material at a time t after mixing with water,

b) determining a proportion of the at least one admixture constituent, preferably at least two admixture constituents, more preferably at least three admixture constituents, in particular four admixture constituents, based on a predefined functional relationship, whereby the predefined functional relationship is defined such that the proportion of the respective admixture constituent is calculable based on the desired controlled introduction of air inputted in step a),

c) making available the proportions of the at least one admixture constituent, preferably at least two admixture constituents, more preferably at least three admixture constituents, in particular four admixture constituents, determined in step b) via a user interface, via a machine interface and/or on a data storage medium.

Inputting of a desired controlled introduction of air in step a) is for example performed by manually inputting the respective data or reading in the respective data via a machine interface. Especially for manually inputting data, a user interface that queries at least the input of desired controlled introduction of air may be provided. In this case, in step a), the desired controlled introduction of air can be captured via a user interface, especially via a graphical user interface. Thereby, in particular, the user interface is configured such that for at least the desired controlled introduction of air a predefined list of selectable parameters is presented to a user.

In particular, the desired controlled introduction of air is a desired content of air, a desired size of air pores, and/or a desired spacing factor of air pores. In particular, the desired controlled introduction of air is a desired content of air. The desired controlled introduction of air can be defined for example according to a desired resistance against freeze-thaw damage. For example according to exposure classes XF1, XF2, XF3, XF4 according to DIN EN 206:2021. However, alternatively or in addition, other exposure parameters might be considered as well.

In particular, the time t after mixing with water is between 0 and 180 minutes. For example the time can be 0 minutes, 20 minutes, 30 minutes, 40 minutes, 60 minutes, 80 minutes, 90 minutes, 120 minutes, 150 minutes, or 180 minutes.

According to embodiments, each of the admixture constituents is associated with its own functional relationship. This allows for deriving the proportions of each admixture constituent in step b) separately.

Preferably, in a computer implemented method of the present invention, the respective proportions are determined in step b) for

- a first admixture constituent which is a polymeric dispersant,
- a second admixture constituent which is an air entraining agent,
- a third admixture constituent which is a first defoaming agent, and
- optionally a fourth admixture constituent which is a second defoaming agent chemically different from the first defoaming agent.

The respective admixture constituents together form one admixture.

According to embodiments, in step b) of the computer implemented method of the present invention, the functional relationship is selected form a list of different predefined multivariable functions with at least two variables.

It can be preferred that the selection of the functional relationship is made depending on the content of unburnt carbon in the fly ash. In other words, in this case the content of unburnt carbon in the fly ash defines

which functional relationship is to be used in step b).

Preferably, the predefined functional relationship is a linear combination of at least two, preferably at least three, more preferably at least four, in particular five terms.

A linear combination is meant to be an expression constructed from a set of terms by multiplying each term by a constant and adding the results. Such relationships turned out to be highly suitable for determining the concentrations of the additives from the parameters considered.

For example, the predefined functional relationship may take the form of:

$$Air_t = a \times C_1 + b \times C_2 + c \times C_3 + d \times C_4 + e$$

where

> Airt is the content of air in Vol-% relative to the total volume of the cementitious material after the time t,
> a, b, c, d, and e are constants, preferably defined by performing a regression analysis on a set of data points comprising the proportions of at least one admixture constituent and optionally the content of unburnt carbon in the fly ash as independent variables and the air content at a time t as dependent variable, and
> $C_1$, $C_2$, $C_3$, and $C_4$ are the proportions of the first, second, third, and fourth admixture constituent respectively.

However, other functional relationships may be used as well.

According to embodiments, in the computer implemented method of the present invention, before step a) the functional relationship is defined, especially with a regression analysis, wherein, in particular, the functional relationship, especially constants of the functional relationship, are defined by performing a regression analysis on a set of data points comprising the proportions of at least one admixture constituent and optionally the content of unburnt carbon in the fly ash as independent variables and the air content at a time t as dependent variable.

However, other approaches for determining the functional relationships can be used as well, e.g. machine learning approaches.

The inventive method can in principle be performed on any kind of computer device and/or control unit. According to a special embodiment, the method is performed on a mobile computer device. In the present context, a mobile computer device in particular is meant to be a handheld computer, i.e. a computer small enough to hold and operate in the hand of a human. Especially selected from a mobile phone, a mobile computer or a portable computer. This allows a user in a concrete batching plant or on a construction site to determine the right proportions of admixture constituents for obtaining a cementitious material with suitably controlled introduction of air for a given application.

In another preferred embodiment, the method is performed within a control unit of a machine that is configured for producing cementitious material, such as e.g. a mixing device for preparing ready mix concrete, precast concrete, for example in a concrete plant, dry mortar, or wet mortar.

Specifically, the method can be implemented in various ways, e.g. in the form of a standalone application running on the mobile device and/or the control unit of a machine without need for any further resources such as for example server systems. This is in particular helpful in areas with limited access to communication networks, e.g. far away from urban centers or underground. However, the method can be implemented as well in a distributed computing environment, e.g. comprising the mobile device and/or the control unit as a client in combination with a dedicated server as a storage unit and/or with a specialized processing unit.

Also, the inventive method can be implemented in a flexible manner with known software architectures, e.g. as native application, a progressive web application (PWA), or a hybrid application (combination of native and PWA). Thereby, helpful internet links to tutorials or support sites as well as sharing functions (e.g. via e-mail, Bluetooth, Airdrop, or others communication means) can be included in such applications as well. Also, the inventive method can be implemented in one single application, or it may be divided into two or more separate applications with appropriate software interfaces for data exchange between the applications. Furthermore, the application(s) can be extended with additional functions in a flexible manner.

Applications can be implemented for any kind of operating systems such as e.g. iOS, Android, Microsoft Windows and/or Linux.

It is possible to repeat a computer implemented method as described above several times with desired controlled introduction of air at different times after mixing. Thereby it is possible to optimize the admixture for a particularly stable introduction of air.

In another aspect, the present invention relates to a non-transitory computer-readable storage medium comprising instruction which, when executed by one or more processors, cause the one or more processors to carry out a method to determine the respective proportions of at least one admixture constituent, preferably at least two admixture constituents, more preferably at least three admixture constituents, in particular four admixture constituents to be added for a controlled introduction of air to a cementitious material comprising a cementitious binder and fly ash, the method comprising the steps of:

> a) inputting a desired controlled introduction of air in the cementitious material at a time t after mixing with water,

b) deriving a proportion of the at least one admixture constituent, preferably at least two admixture constituents, more preferably at least three admixture constituents, in particular four admixture constituents, based on a predefined functional relationship, whereby the predefined functional relationship is defined such that the proportion of the respective constituent is calculable based on the desired air content inputted in step a),

c) making available the proportions of the at least one admixture constituent, preferably at least two admixture constituents, more preferably at least three admixture constituents, in particular four admixture constituents, determined in step b) via a user interface, via a machine interface and/or on a data storage medium.

It is further possible, to manufacture an admixture from the admixture constituents in the proportions determined by a computer-implemented method of the present invention. Therefore, the present invention also relates to a computer implemented method to determine the respective proportions of at least one admixture constituent, preferably at least two admixture constituents, more preferably at least three admixture constituents, in particular four admixture constituents to be added for a controlled introduction of air to a cementitious material comprising a cementitious binder and fly ash, the method comprising the steps of:

a) inputting a desired controlled introduction of air in the cementitious material at a time t after mixing with water,
b) deriving a proportion of the at least one admixture constituent, preferably at least two admixture constituents, more preferably at least three admixture constituents, in particular four admixture constituents, based on a predefined functional relationship, whereby the predefined functional relationship is defined such that the proportion of the respective admixture constituent is calculable based on the desired controlled introduction of air inputted in step a),
c) making available the proportions of the at least one admixture constituent, preferably at least two admixture constituents, more preferably at least three admixture constituents, in particular four admixture constituents, determined in step b) via a user interface, via a machine interface and/or on a data storage medium,
d) manufacturing an admixture, where said manufacturing comprises the steps of

d1) providing the individual admixture constituents,
d2) optionally premixing the individual admixture constituents,
d3) optionally assembling the premixed admixture constituents, for example in one or more containers,
d4) intergrinding and/or intermixing intermixing the individual admixture constituents or the premix of individual admixture constituents with a cementitious material.

Embodiments and features as described above also apply to this aspect.

**Brief description of the drawings**

The drawings used to explain the embodiments show:

Fig. 1 a flow diagram of an inventive computer implemented method to determine the respective proportions of four different constituent of an admixture.

**Exemplary embodiments**

Example 1 - Method for the controlled introduction of air

A cementitious composition was produced by dry mixing 840 g of Ordinary Portland Cement (CEM I), 358 g of fly ash (methylene blue value 0.37 mg/g), and 2402 g of washed land sand. Thereto, 564 g of mixing water were added at 20 °C to realize a water to binder ratio of 0.47. Together with the mixing water a polymeric dispersant (3 w% rel. to the cementitious binder), an air entraining agent (0.01 w% rel. to the cementitious binder), and two different defoaming agents (in a combined amount of 0.14 w% rel. to the cementitious binder) were intermixed in a Hobart mixer. Air content was measured according to JIS A 1128:2019 directly after mixing to be 3.4 Vol-%, 3.5 Vol-% 30 minutes after mixing, and 3.4 w% 60 minutes after mixing.

The polymeric dispersant used was as described above with the monomer I having $R^1$ = H, $R^5$ = H, n = 90, $A_1 = C_2H_4$; the monomer II having $R^5$ = p-t-Bu, $R^2$, $R^3$ = H, n = 6, $A_1 = C_2H_4$; the monomer III having m = 3, n = 3, $R^4$ = H, $A_1$, $A_2 = C_2H_4$; monomer V being formaldehyde. The molar ratio of a : b : c : d = 1 : 2: 0.5 : 0. The molar ratio (a+b+c) : e = 5. The molecular weight measured by GPC being 23000 g/mol.

The air entraining agent used was sodium lauryl ether sulfate (as 35w% solution in water).

The defoaming agents used were a 1:1 mixture of polypropylene oxide)stearyl ether (as 70 w% solution in water) and poly(block-propylene oxide-ethylene oxide) lauryl ether.

Example 2 - Computer implemented method to determine the respective proportions of admixture constituents

Fig. 1 shows a flow diagram of an inventive computer implemented method 10 to determine the respective proportions of four admixture constituents to be added for a controlled introduction of air to a cementitious material comprising a cementitious binder and fly ash. Thereby, apart from the four different constituents of the admixture to be added, the cementitious material is predefined.

In a first step 11, a desired controlled introduction of air is inputted, e.g. via a graphical user interface that is configured such that a predefined list of selectable items is presented to a user.

The desired controlled introduction of air is a desired content of air, a desired size of air pores, and/or a desired spacing factor of air pores. Any of these inputs can be automatically transformed to numerical values during step 11.

In the second step 12, for each of the four constituents of an admixture, a proportion of the respective constituent is calculated based on a predefined functional relationship FR. The predefined functional relationships FR, which have been determined in advance by testing, are for example stored in a memory unit M and are represented by formulas of type Airt = a $\times$ $C_1$ + b $\times$ $C_2$ + c $\times$ $C_3$ + d $\times$ $C_4$ + e where

Airt is the content of air in Vol-% relative to the total volume of the cementitious material after the time t,

a, b, c, d, and e are constants, preferably defined by performing a regression analysis on a set of data points comprising the proportions of at least one admixture constituent and optionally the content of unburnt carbon in the fly ash as independent variables and the air content at a time t as dependent variable, and

$C_1$, $C_2$, $C_3$, and $C_4$ are the proportions of the first, second, third, and fourth admixture constituent respectively.

Specifically, in a first sub-step 12.1, the proportion $c_1$ of a first constituent $C_1$, especially a polymeric dispersant, is determined by selecting a predefined functional relationship. Likewise, the proportion $c_2$ of a second constituent $C_2$, especially an air-entrainer, is calculated in a second sub-step 12.2, the proportion $c_3$ of a third constituent $C_3$, especially a first defoaming agent, is calculated in a third sub-step 12.3, and the proportion $c_4$ of a fourth constituent $C_4$, especially a second defoaming agent, is calculated in a fourth sub-step 12.4.

In a third step 13, the concentrations $c_1$, $c_2$, $c_3$, and $c_4$ of the additives $C_1$, $C_2$, $C_3$, and $C_4$ are made available, e.g. via a user interface, for example a display.

Thereafter, a user can prepare a cementitious material with the desired controlled introduction of air, by intergrinding and/or intermixing the respective constituents of the admixture with the cementitious material.

## Claims

1. A method for the controlled introduction of air in a cementitious material comprising a cementitious binder and fly ash, said method comprising the steps of:

   i) providing a cementitious material comprising fly ash,
   ii) providing an admixture comprising or consisting of a polymeric dispersant, at least one air-entraining agent, at least one anti-foam agent, and water, and ii) intergrinding and/or intermixing said cementitious material and said admixture.

2. The method as claimed in claim 1, **characterized in that** the polymeric dispersant is a phenol resin-based dispersant obtained from the polycondensation of the following monomers (i) to (v):

   (i) a mol% of monomers of formula (I)

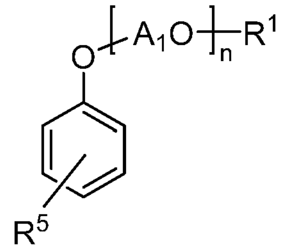

(I),

   (ii) b mol% of monomers of formula (II)

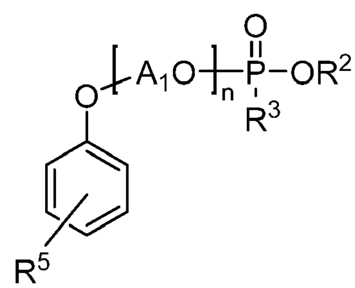

(II),

   (iii) c mol% of monomers of formula (III)

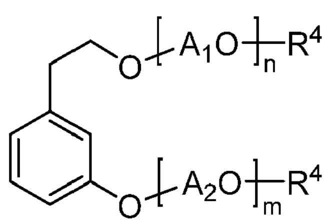

(III),

and

(iv) d mol% of monomers of formula (IV)

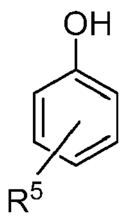

(IV),

(v) e mol% of monomers selected from aldehydes, preferably formaldehyde, metaformaldehyde, paraformaldehyde, formalin, or mixtures thereof,

wherein

$R^1$ is H, a C1 - C4 alkyl group, or a C2 - C5 acyl group,
$R^2$ is H, an alkali metal or an alkaline earth metal ion, or a moiety of structure

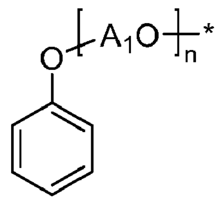

,

$R^3$ is H or is O-$R^2$,
$R^4$ is H, a C1 - C4 alkyl group, or a phosphate ester group,
$R^5$ is H, a C1 - C18 alkyl group, a polyisobutylene moiety, or a sulfonic acid moiety,
$A_1$ and $A_2$ independently of one another are $C_xH_{2x}$ group with x = 2 to 5,
n = 1 - 350, m = 2 - 300,
a molar ratio of a : b : c : d is 0.1 - 2.0 : 0.1 - 6.0 : 0.1 - 2.0 : 0.0 - 0.5, and a molar ratio of (a + b + c + d) : e is from 1 : 10 to 10 : 1.

**3.** The method as claimed in at least one of the preceding claims, **characterized in that** the fly ash has a content of unburnt carbon of between 0.01 - 12.0 w%, preferably 0.1 - 10.0 w%, more preferably 0.1 - 6.0 w%, relative to the total dry weight of the fly ash.

**4.** The method as claimed in at least one of the preceding claims, **characterized in that** the fly ash is according to standard JIS 6201:2015.

**5.** The method as claimed in at least one of the preceding claims, **characterized in that** the content of fly ash in the cementitious material is not more than 70 w%, preferably not more than 50 w%, more preferably not more than 30 w%, relative to the total dry weight of the cementitious material.

**6.** The method as claimed in at least one of the preceding claims, **characterized in that** the admixture is intergrinded and/or is intermixed with the cementitious material in an amount so that the following weight ratios result (in each case relative to the total weight of cementitious binder contained):

- 0.1 - 5 w%, preferably 0.5 - 3 w%, especially 1 - 2 w%, of the polymeric dispersant,
- 0.001 - 0.03 w%, preferably 0.005 - 0.02 w%, especially 0.01 - 0.015 w%, of the at least one air-entraining agent,
- 0.01 - 0.5 w%, preferably 0.02 - 0.25 w%, especially 0.1 - 0.2 w%, of the at least one anti-foam agent.

**7.** A cementitious material obtained by a method as claimed in at least one of claims 1 - 6.

**8.** The cementitious material according to claim 7, **characterized in that** it contains an amount of air between 1 - 20 Vol-%, preferably between 3-10 Vol-%, more preferably between 4-8 Vol-%, relative to the total volume of the cementitious material at a time $t_0$ after mixing with water.

**9.** The cementitious material according to at least one of claims 7 or 8, **characterized in that** the amount of air in the cementitious material at a time $t_1$ after mixing with water differs by not more than 100%, preferably not more than 33%, more preferably not more than 20%, especially not more than 10%, from the amount of air at a time $t_0$ after mixing with water, where the time $t_1$ is later than the time $t_0$.

**10.** An admixture for use in a method as claimed in at least one of claims 1 - 6, said admixture comprising (in each case relative to the total weight of the admixture):

a) 1 - 20 w%, preferably 5 - 15 w%, more preferably 8-13 w%, of a polymeric dispersant,
b) 1 - 20 w% of at least one air-entraining agent,
c) 0.1 - 10 w% of at least one anti-foaming agent, and
d) the remainder to 100 w% of water.

**11.** A computer implemented method to determine the respective proportions of at least one admixture constituent, preferably at least two admixture constituents, more preferably at least three admixture constituents, in particular four admixture constituents to be added for a controlled introduction of air to a cementitious material comprising a cementitious binder and fly ash, the method comprising the steps of:

a) inputting a desired controlled introduction of air in the cementitious material at a time t after mixing with water,

b) deriving a proportion of the at least one admixture constituent, preferably at least two admixture constituents, more preferably at least three admixture constituents, in particular four admixture constituents, based on a predefined functional relationship, whereby the predefined functional relationship is defined such that the proportion of the respective admixture constituent is calculable based on the desired controlled introduction of air inputted in step a),

c) making available the proportions of the at least one admixture constituent, preferably at least two admixture constituents, more preferably at least three admixture constituents, in particular four admixture constituents, determined in step b) via a user interface, via a machine interface and/or on a data storage medium.

**12.** The method as claimed in claim 11, **characterized in that** the respective proportions are determined in step b) for

- a first admixture constituent which is a polymeric dispersant,
- a second admixture constituent which is an air entraining agent,
- a third admixture constituent which is a first defoaming agent, and
- optionally a fourth admixture constituent which is a second defoaming agent chemically different from the first defoaming agent.

**13.** The method as claimed in at least one of claims 11 - 12, **characterized in that** in step b) the functional relationship is selected form a list of different predefined multivariable functions with at least two variables.

**14.** The method as claimed in at least one of the claims 11 - 13, **characterized in that** before step a) the functional relationship is defined, especially with a regression analysis, wherein, in particular, the functional relationship, especially constants of the functional relationship, are defined by performing a regression analysis on a set of data points comprising the proportions of at least one admixture constituent and optionally the content of unburnt carbon in the fly ash as independent variables and the air content at a time t as dependent variable.

**15.** The method as claimed in at least one of the claims 11 - 14, **characterized in that** the predefined functional relationship is a linear combination of at least two, preferably at least three, more preferably at least four, in particular five terms.

**16.** A non-transitory computer-readable storage medium comprising instruction which, when executed by one or more processors, cause the one or more processors to carry out the method as claimed in at least one of claims 11 - 15.

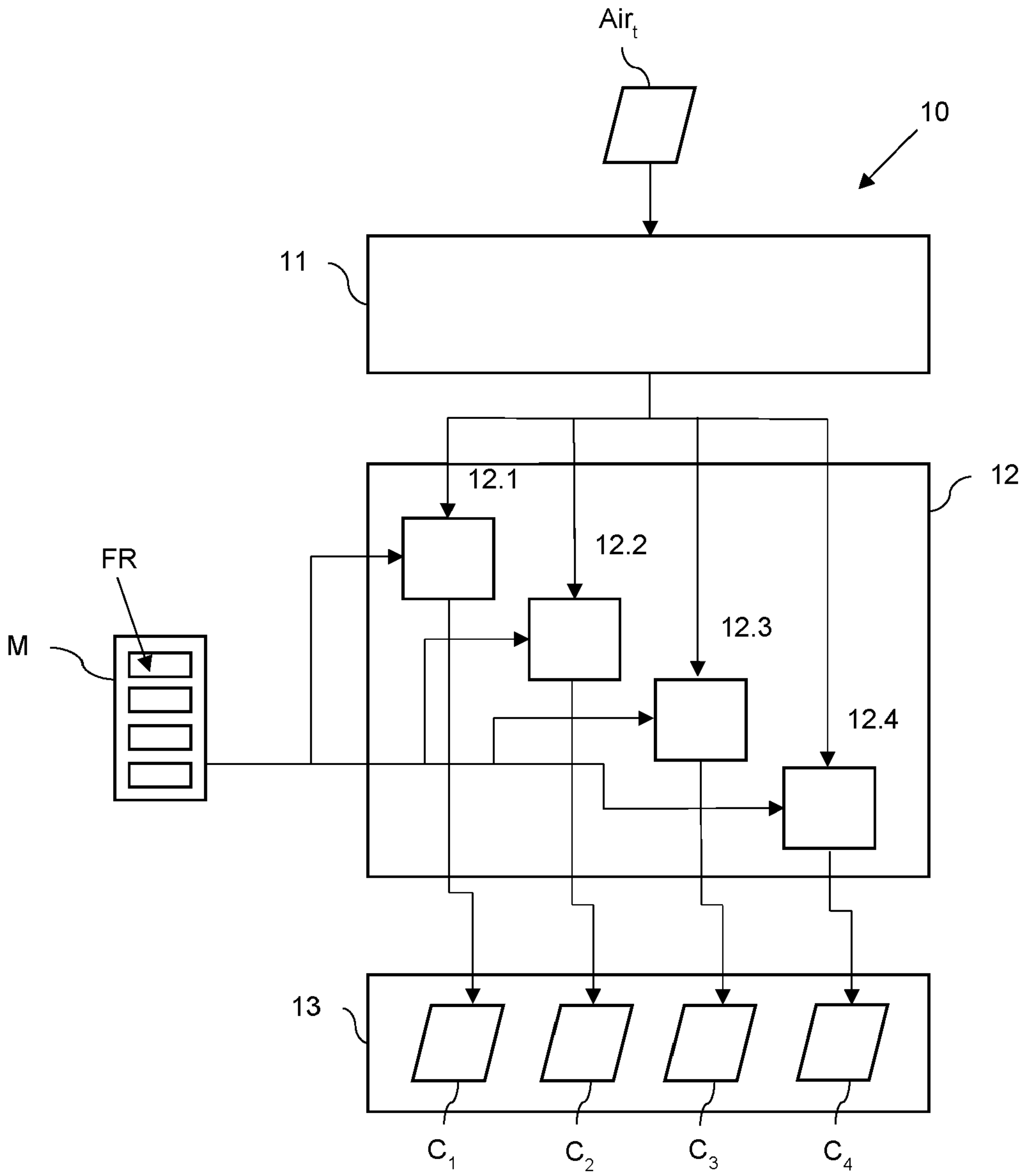


Figure 1

Europäisches Patentamt
European Patent Office
Office européen des brevets

# EUROPEAN SEARCH REPORT

Application Number
EP 23 21 2125

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 135 650 A1 (SIKA TECH AG [CH]) 1 March 2017 (2017-03-01) * paragraphs [0004] - [0040] * | 1,3-10 | INV. C04B20/02 C04B28/02 C04B40/00 G01N33/38 G06Q50/08 |
| X | WO 01/42162 A1 (MBT HOLDING AG [CH]) 14 June 2001 (2001-06-14) * the whole document * | 1,3-10 | |
| X | WO 2021/102294 A1 (GCP APPLIED TECH INC [US]) 27 May 2021 (2021-05-27) * the whole document * | 1,3-10 | |
| X | EP 3 581 548 A1 (SIKA TECH AG [CH]; TOHO CHEMICAL IND CO LTD [JP]) 18 December 2019 (2019-12-18) * the whole document * | 1-10 | |
| A | WO 2004/067471 A2 (HANDY CHEMICALS LTD [CA]; HILL RUSSELL [US] ET AL.) 12 August 2004 (2004-08-12) * the whole document * | 1-10 | TECHNICAL FIELDS SEARCHED (IPC) |
| X | US 2006/039233 A1 (FARRINGTON STEPHEN A [US] ET AL) 23 February 2006 (2006-02-23) * the whole document * | 11-16 | C04B G01N G06Q |
| X | US 2022/253734 A1 (AGHDASI PARHAM [US]) 11 August 2022 (2022-08-11) * the whole document * | 11-16 | |
| X | US 7 398 131 B2 (NOMADICS INC [US]) 8 July 2008 (2008-07-08) * the whole document * | 11-16 | |

The present search report has been drawn up for all claims

| Place of search | Date of completion of the search | Examiner |
|---|---|---|
| Munich | 23 August 2024 | Gattinger, Irene |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 23 21 2125

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 23 21 2125

The Search Division considers that the present European patent application does not comply with the requirements of unity of invention and relates to several inventions or groups of inventions, namely:

1. claims: 1-10

Method for controlled introduction of air and admixture therefore

---

2. claims: 11-16

Computer implemented method for determination of admixture proportions and non-transitory computer-readable storage medium

---

# ANNEX TO THE EUROPEAN SEARCH REPORT ON EUROPEAN PATENT APPLICATION NO. EP 23 21 2125

This annex lists the patent family members relating to the patent documents cited in the above-mentioned European search report. The members are as contained in the European Patent Office EDP file on
The European Patent Office is in no way liable for these particulars which are merely given for the purpose of information.

23-08-2024

| Patent document cited in search report | Publication date | Patent family member(s) | Publication date |
|---|---|---|---|
| EP 3135650 A1 | 01-03-2017 | CN 106458752 A | 22-02-2017 |
| | | CN 106573840 A | 19-04-2017 |
| | | EP 3135650 A1 | 01-03-2017 |
| | | JP 6621737 B2 | 18-12-2019 |
| | | JP 6629723 B2 | 15-01-2020 |
| | | JP WO2015163468 A1 | 20-04-2017 |
| | | JP WO2015163469 A1 | 18-05-2017 |
| | | TW 201546016 A | 16-12-2015 |
| | | US 2017044063 A1 | 16-02-2017 |
| | | WO 2015163468 A1 | 29-10-2015 |
| | | WO 2015163469 A1 | 29-10-2015 |
| WO 0142162 A1 | 14-06-2001 | AT E298733 T1 | 15-07-2005 |
| | | AU 2005801 A | 18-06-2001 |
| | | CA 2393625 A1 | 14-06-2001 |
| | | DE 60021115 T2 | 11-05-2006 |
| | | EP 1242330 A1 | 25-09-2002 |
| | | JP 2003516301 A | 13-05-2003 |
| | | MX 238784 B | 19-07-2006 |
| | | US 2002107310 A1 | 08-08-2002 |
| | | WO 0142162 A1 | 14-06-2001 |
| WO 2021102294 A1 | 27-05-2021 | CA 3162491 A1 | 27-05-2021 |
| | | US 2023032346 A1 | 02-02-2023 |
| | | WO 2021102294 A1 | 27-05-2021 |
| EP 3581548 A1 | 18-12-2019 | CN 110520395 A | 29-11-2019 |
| | | EP 3581548 A1 | 18-12-2019 |
| | | JP 7069053 B2 | 17-05-2022 |
| | | JP WO2018147378 A1 | 20-02-2020 |
| | | RU 2019127901 A | 09-03-2021 |
| | | US 2020055785 A1 | 20-02-2020 |
| | | WO 2018147378 A1 | 16-08-2018 |
| WO 2004067471 A2 | 12-08-2004 | AU 2004207782 A1 | 12-08-2004 |
| | | BR PI0406927 A | 07-02-2006 |
| | | CA 2514190 A1 | 12-08-2004 |
| | | CN 1741864 A | 01-03-2006 |
| | | CN 1761632 A | 19-04-2006 |
| | | EP 1611069 A2 | 04-01-2006 |
| | | EP 2266929 A2 | 29-12-2010 |
| | | ES 2355201 T3 | 23-03-2011 |
| | | JP 2006516529 A | 06-07-2006 |
| | | KR 20050092447 A | 21-09-2005 |
| | | MY 141254 A | 31-03-2010 |
| | | NZ 541478 A | 31-03-2009 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 23 21 2125

This annex lists the patent family members relating to the patent documents cited in the above-mentioned European search report. The members are as contained in the European Patent Office EDP file on
The European Patent Office is in no way liable for these particulars which are merely given for the purpose of information.

23-08-2024

| Patent document cited in search report | | Publication date | Patent family member(s) | Publication date |
|---|---|---|---|---|
| | | | US 2004206276 A1 | 21-10-2004 |
| | | | US 2009199742 A1 | 13-08-2009 |
| | | | US 2009199743 A1 | 13-08-2009 |
| | | | US 2009199744 A1 | 13-08-2009 |
| | | | WO 2004067471 A2 | 12-08-2004 |
| US 2006039233 | A1 | 23-02-2006 | NONE | |
| US 2022253734 | A1 | 11-08-2022 | NONE | |
| US 7398131 | B2 | 08-07-2008 | US 2007179653 A1 | 02-08-2007 |
| | | | US 2008221815 A1 | 11-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018147378 A